(19) **Europäisches Patentamt**
**European Patent Office**
**Office européen des brevets**

(11) **EP 3 199 150 A1**

(12) # EUROPEAN PATENT APPLICATION
published in accordance with Art. 153(4) EPC

(43) Date of publication:
**02.08.2017 Bulletin 2017/31**

(21) Application number: **15843255.9**

(22) Date of filing: **24.09.2015**

(51) Int Cl.:
*A61K 31/05* [(2006.01)]   *A61K 9/107* [(2006.01)]
*A61K 47/12* [(2006.01)]   *A61K 47/18* [(2017.01)]
*A61K 47/20* [(2006.01)]   *A61K 47/24* [(2006.01)]
*A61P 23/00* [(2006.01)]

(86) International application number:
**PCT/JP2015/076870**

(87) International publication number:
**WO 2016/047664 (31.03.2016 Gazette 2016/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **25.09.2014 JP 2014195096**

(71) Applicant: **Fujifilm Corporation
Minato-ku
Tokyo 106-8620 (JP)**

(72) Inventors:
• **SAKATA Yoshinori**
  **Ashigarakami-gun**
  **Kanagawa 258-8577 (JP)**
• **IZUMI Yasuyuki**
  **Ashigarakami-gun**
  **Kanagawa 258-8577 (JP)**
• **TSUJIHATA Shigetomo**
  **Ashigarakami-gun**
  **Kanagawa 258-8577 (JP)**

(74) Representative: **Hoffmann Eitle
Patent- und Rechtsanwälte PartmbB
Arabellastraße 30
81925 München (DE)**

(54) **PROPOFOL-CONTAINING OIL-IN-WATER EMULSION COMPOSITION AND METHOD FOR PRODUCING SAME**

(57)     Provided are a propofol-containing oil-in-water emulsion composition having high stability even in a case in which the emulsion composition is charged into a plastic container; and a method for producing the same. Provided is a propofol-containing oil-in-water emulsion composition including at least one selected from the group consisting of trishydroxymethylaminomethane, phosphoric acid, and triethanolamine; propofol; an oily component; an emulsifier; and water, in which a dissolved oxygen concentration is 5 mg/L or less and an average emulsion particle size is 180 nm or less.

**EP 3 199 150 A1**

**Description**

**BACKGROUND OF THE INVENTION**

1. Field of the Invention

The present invention relates to a propofol-containing oil-in-water emulsion composition having excellent stability, and a method for producing the same.

2. Description of the Related Art

Propofol (chemical name: 2,6-diisopropylphenol) has features such as rapid induction of anesthesia, rapid awakening, and less unpleasantness such as nausea and vomiting after awakening, and is widely used as an intravenous general anesthetic and an intravenous sedative for the purpose of sedation during surgery and in intensive care units or the like.

JP2005-538191A describes an aqueous pharmaceutical composition including propofol and one or more excipients, and more specifically, an aqueous propofol formulation including a block copolymer, polyethylene glycol (PEG), and 2,6-diisopropylphenol is described.

JP2009-504634A describes an aqueous anesthetic composition appropriate for parenteral administration, which includes propofol, 2-hydroxypropyl-β-cyclodextrin, and lignocaine as a local anesthetic, or a salt thereof with an acid.

JP2010-534555A describes a composite type emulsifier including two kinds or two or more kinds of emulsifiers selected from the group consisting of 10% w/v or less of phospholipids, 30% w/v or less of a PEG-based emulsifier, and 10% w/v or less of a poloxamer-based substance, the indicated proportions being contents in an emulsion. In Example 25 of the same patent document, a propofol emulsion is described.

**SUMMARY OF THE INVENTION**

The formulation of JP2005-538191A is an aqueous propofol formulation, and therefore, it is necessary to use a large amount of surfactants, while there is a risk of adverse side effects and toxicity. Furthermore, in the Examples of JP2005-538191A, stability in glass containers is evaluated; however, in regard to the case in which the aqueous propofol formulation of JP2005-538191A is charged into a plastic container, in which a preparation charged therein is likely to become unstable compared to a glass container, it is speculated that the formulation is not sufficiently stable.

The composition described in JP2009-504634A is also an aqueous anesthetic composition, and since cyclodextrin is used in large quantities, there is a risk of adverse side effects and toxicity. Furthermore, since the stability evaluation in the Examples of JP2009-504634A is also performed using glass vials, it is not clearly known whether the composition is sufficiently stable, particularly in a case in which the composition is charged into a plastic container. Furthermore, according to JP2009-504634A, the effects of an antioxidant and a buffering agent are not clearly disclosed, and it is only described that the stability of the preparation is improved by maintaining the composition to be weakly acidic.

The propofol emulsion described in Example 25 of JP2010-534555A includes tocopherol (VE) as an antioxidant (Formulations 2 and 4 in Table 16 of JP2010-534555A); however, since large amounts of emulsifiers are used, there is a risk of adverse side effects and toxicity. Furthermore, an evaluation was performed for 10 days at 40°C in order to check stability of the propofol emulsion; however, there is no description on stability of propofol itself, and it is speculated that the propofol emulsion is not sufficiently stable.

Propofol has low stability to heat or low stability over time, and stability thereof may be markedly decreased depending on the type of the container. Thus, it is an object of the invention to provide a propofol-containing oil-in-water emulsion composition that has no risk of adverse side effects or toxicity and is highly stable even in a case in which the emulsion composition is charged into a plastic container, and a method for producing the same.

The inventors of the invention conducted a thorough investigation in order to solve the problems described above, and as a result, the inventors found that when at least one selected from the group consisting of trishydroxymethylaminomethane, phosphoric acid, and triethanolamine is incorporated into a propofol-containing oil-in-water emulsion composition, the dissolved oxygen concentration is adjusted to be 5 mg/L or less, and the average emulsion particle size is adjusted to be 180 nm or less, a propofol-containing oil-in-water emulsion composition having high stability even in a case in which the emulsion composition is charged into a plastic container, can be provided. Thus, the inventors completed the invention.

That is, according to the invention, the following inventions are provided.

(1) A propofol-containing oil-in-water emulsion composition comprising: at least one selected from the group consisting of trishydroxymethylaminomethane, phosphoric acid, and triethanolamine; propofol; an oily component; an emulsifier; and water, in which a dissolved oxygen concentration is 5 mg/L or less and an average emulsion particle size is 180 nm or less.

(2) The propofol-containing oil-in-water emulsion composition according to (1), which is charged into a plastic container.

(3) The propofol-containing oil-in-water emulsion composition according to (1) or (2), in which the at least one selected from the group consisting of trishydroxymethylaminomethane, phosphoric acid, and triethanolamine is trishydroxymethylaminomethane.

(4) The propofol-containing oil-in-water emulsion composition according to any one of (1) to (3), further comprising an antioxidant, in which the antioxidant is at least one selected from the group consisting of citric acid or a salt thereof, ascorbic acid or a salt thereof, and a thiol-based antioxidant.

(5) The propofol-containing oil-in-water emulsion composition according to (4), in which the antioxidant is a thiol-based antioxidant.

(6) The propofol-containing oil-in-water emulsion composition according to (4) or (5), in which the trishydroxymethylaminomethane, and the thiol-based antioxidant and the citric acid or a salt thereof as antioxidants are included.

(7) The propofol-containing oil-in-water emulsion composition according to (4) or (5), in which the trishydroxymethylaminomethane, and the thiol-based antioxidant and the ascorbic acid or a salt thereof as antioxidants are included.

(8) The propofol-containing oil-in-water emulsion composition according to any one of (4) to (7), in which the thiol-based antioxidant is cysteine or a salt thereof, thioglycolic acid or a salt thereof, or thioglycerol.

(9) The propofol-containing oil-in-water emulsion composition according to any one of (1) to (8), in which the propofol at a proportion of 0.1 to 5 w/v%, the emulsifier at a proportion of 0.1 to 2 w/v%, and the oily component at a proportion of 0.1 to 9.9 w/v% are included.

(10) A method for producing the propofol-containing oil-in-water emulsion composition according to any one of (1) to (9), the method comprising: a step of treating a composition including propofol, an oily component, an emulsifier, and water using a high pressure homogenizer under the conditions of a pressure of 200 MPa or more; and a step of sterilizing the composition thus obtained by passing the composition through a filter having a pore size of 0.22 μm or less.

(11) The production method according to (10), in which the material for the filter is cellulose acetate.

According to the invention, a propofol-containing oil-in-water emulsion composition having high stability even in a case in which the emulsion composition is charged into a plastic container, and a method for producing the same are provided.

## DESCRIPTION OF THE PREFERRED EMBODIMENTS

Hereinafter, embodiments of the invention will be explained in detail.

According to the present specification, the propofol-containing oil-in-water emulsion composition may be simply referred to as "emulsion composition" or "composition".

According to the present specification, in a case in which the amount of each component in the composition is mentioned, and in a case in which a plurality of substances corresponding to each component are present in the composition, unless particularly stated otherwise, the amount means the sum total amount of the plurality of substances present in the composition.

Regarding the term "step" as used in the present specification, not only independent steps are included in the present term; but also, even in a case in which a step cannot be clearly distinguished from another step, the relevant step is to be included in the present term as long as the step is capable of achieving a predetermined effect of the present step.

Furthermore, according to the present specification, a numerical value range indicated using the symbol "~" represents a range including the numerical values described before and after the symbol "~" as the minimum value and the maximum value, respectively.

According to the invention, for example, the unit "w/v%" used for the amount of incorporation (concentration) of each component that constitutes the emulsion composition of the invention means the percentage of the mass (g) of each component with respect to the volume (capacity) of 100 mL of the whole composition (following Expression 1a). In a case in which the amount of incorporation is expressed as the mass with respect to the total capacity of the composition, unless particularly stated otherwise, the unit "w/v%" similarly means mass (g) of each component with respect to a volume (capacity) of 100 mL of the whole composition. For example, the amount of incorporation of a component that is incorporated at a mass of 1.0 g into 100 mL of the composition is described as "1.0 w/v%".

Furthermore, according to the invention, for example, the unit "w/w%" used for the amount of incorporation (concentration) of each component that constitutes the emulsion composition of the invention means the percentage of the mass (g) of each component with respect to the mass (g) of a reference substance (following Expression 1b).

Expression 1a: [Each component mass (g) / volume (capacity) of 100 mL of whole composition] × 100 (%)

Expression 1b: [Mass (g) of each component / mass (g) of reference substance] × 100 (%)

In the following description, the invention will be explained.

The propofol-containing oil-in-water emulsion composition of the invention is a propofol-containing oil-in-water emulsion composition including at least one selected from the group consisting of trishydroxymethylaminomethane, phosphoric acid, and triethanolamine; propofol; an oily component; an emulsifier; and water, and having a dissolved oxygen concentration of 5 mg/L or less and an average emulsion particle size of 180 nm or less.

When the emulsion composition of the invention is used, since the emulsion composition is expected to have high oxygen permeability, high stability of propofol can be achieved even in a plastic container that is disadvantageous in terms of the stability of propofol. According to the invention, it has been demonstrated that when at least one selected from the group consisting of trishydroxymethylaminomethane, phosphoric acid, and triethanolamine is added as a buffering agent, and the threshold value of the dissolved oxygen concentration is defined to be 5 mg/L or less, stability of propofol is dramatically enhanced.

An oil-in-water emulsion refers to a state in which finely dispersed liquid droplets of an oil phase are surrounded by emulsifier molecules, and the outer phase is an aqueous continuous phase. The aqueous component and the oily component are stably mixed by the action of the emulsifier.

The dissolved oxygen concentration in the propofol-containing oil-in-water emulsion composition of the invention is 5 mg/L or less. When the dissolved oxygen concentration is adjusted to the range described above, even after a long lapse of time, suppression of decomposition of propofol can be achieved. The dissolved oxygen concentration is preferably 1 mg/L or less, more preferably 0.1 mg/L or less, and even more preferably 0.01 mg/L or less. The lower limit of the dissolved oxygen concentration is not particularly limited; however, the lower limit may be 0 mg/L or more, or may be 0 mg/L.

The method for measuring the dissolved oxygen concentration can be measured by a diaphragm polarographic method. In the diaphragm polarographic method, a voltage required for reducing oxygen that has penetrated through a diaphragm is applied between a working electrode and a counter electrode using an external constant voltage apparatus. In a case in which KCl is used for an internal liquid, and Ag/AgCl is used for the counter electrode, the potential of the counter electrode does not change even if some current flows. That is, since polarization does not occur, a constant applied voltage is always applied to the working electrode. As a result of application of a certain applied voltage to the working electrode, a current proportional to the oxygen concentration flows. The dissolved oxygen concentration can be measured using a commercially available dissolved oxygen sensor, for example, an INLAB (registered trademark) 605 dissolved oxygen sensor connected to SEVEN GOPRO (manufactured by Mettler Toledo International, Inc.).

In regard to the measurement, regarding the temperature and pressure, it is preferable to perform measurement at normal temperature and atmospheric pressure, and it is more preferable to perform measurement at a temperature between 20°C and 30°C, and a pressure of atmospheric pressure.

The average emulsion particle size of the propofol-containing oil-in-water emulsion composition of the invention is 180 nm or less, preferably 160 nm or less, and more preferably 140 nm or less. When the average emulsion particle size is adjusted to be in the range described above, sterilization by filtration is enabled, and autoclave (heat)-induced decomposition of propofol can be suppressed. The lower limit of the average emulsion particle size is not particularly limited; however, the lower limit is generally 30 nm or more, and preferably 50 nm or more.

The average emulsion particle size can be made smaller by changing the conditions or composition employed for fine emulsification. In a case in which a fine emulsification process is carried out using a high pressure homogenizer, the average particle size can be made small by further increasing the pressure and increasing the frequency of passages. In order to adjust the average emulsion particle size of the propofol-containing oil-in-water emulsion composition of the invention to be 180 nm or less, it can be achieved by adjusting the amount of the emulsifier to 15 w/w% or more with respect to the oily component.

The particle size of an emulsion can be measured using a commercially available particle size distribution analyzer or the like. Regarding the particle size distribution analysis method, optical microscopy, confocal laser microscopy, electron microscopy, atomic force microscopy, static light scattering, laser diffraction, dynamic light scattering, centrifugal sedimentation, electric pulse measurement, chromatography, ultrasonic attenuation, and the like are known, and apparatuses corresponding to the respective principles are commercially available. For the measurement of the emulsion particle size according to the invention, it is preferable to use dynamic light scattering, from the viewpoints of the particle size range and the ease of measurement. Examples of commercially available measuring apparatus using dynamic light scattering include NANOTRAC UPA (Nikkiso Co., Ltd.), dynamic light scattering type particle size distribution analyzer LB-550 (Horiba, Ltd.), concentrated system particle size analyzer FPAR-1000 (Otsuka Electronics Co., Ltd.).

In regard to the average emulsion particle size according to the invention, the median diameter of a scattering intensity distribution obtainable by a Contin method using a dynamic light scattering particle size distribution analyzer was designated as the average emulsion particle size.

The propofol-containing oil-in-water emulsion composition of the invention can be provided in the form of being contained in a container. The material for the container is not particularly limited, and any of a plastic container or a glass container may be used; however, a container preferable from the viewpoint of having less risk of damage and being lightweight is a plastic container. The plastic container may be any one of a plastic syringe, a plastic ample, a plastic vial, and a plastic bag; however, a preferred plastic container is a plastic syringe.

In a case in which the container is a plastic syringe, an emulsion composition that has been sterilized by filtration may be charged through the tip where an injection needle is mounted, and then the tip may be tightly sealed with a rubber or plastic part. Also, an emulsion composition that has been sterilized by filtration may be charged through the plunger rod part, and then the plunger rod part may be tightly sealed with a rubber or plastic gasket, or a plunger rod. A plastic syringe filled with an emulsion composition as such can be preferably used as a prefilled syringe preparation, from the viewpoints of convenience and prevention of erroneous operation.

In a case in which the container is a plastic ample, the ample can be produced by a so-called blow-fill-seal system, by which a plastic container is molded, simultaneously the container is filled with an emulsion composition, and the container is sealed immediately after filling. An ample produced by such a blow-fill-seal system is preferably made of a semi-hard plastic.

In a case in which the container is a plastic vial, the preparation can be produced by dispensing an emulsion composition that has been sterilized by filtration into a vial, and sealing the opening by means of a rubber stopper and if desired, an aluminum cap in combination with the stopper.

In a case in which the container is a plastic bag, the preparation can be produced by a method of aseptically filling a separately sterilized plastic bag with an emulsion composition that has been sterilized by filtration, and sealing the plastic bag; a method of filling a bag with an emulsion composition, sealing the bag, and then sterilizing the container together with the content according to a conventional method (for example, a high pressure steam sterilization method, a hot water immersion sterilization method, or a hot water shower sterilization method); or the like.

Propofol is a general name of 2,6-diisopropylphenol, and for example, as described in JP2002-179562A, it is known that propofol can be utilized as a general anesthetic, a sedative, or the like in the pharmaceutical field. Regarding the solubility of propofol in water, in a case in which propofol is used in its effective dose, the solubility is fairly low compared to other drugs in the same effective dose. In the emulsion composition of the invention, propofol is present in an amount of 0.1 w/v% to 5 w/v%, preferably in the amount of 0.1 w/v% to 2 w/v%, and more preferably 0.5 w/v% to 2 w/v%, with respect to the capacity of the whole emulsion composition.

Furthermore, according to the invention, the mass of propofol with respect to the mass of the oily component is preferably 1 w/w% to 50 w/w%, and more preferably 5 w/w% to 35 w/w%.

The emulsion composition of the invention includes an oily component.

The "oily component" according to the invention means, in a wide sense, a component that is pharmaceutically acceptable and can constitute an oil phase in an oil-in-water emulsion composition. Examples of the oily component according to the invention include medium-chain fatty acid triglycerides; plant oils (that is, natural triglycerides), chemically synthesized triglycerides, or long-chain fatty acid triglycerides such as animal oils; mineral oils; synthetic oils; refined oils; ester oils; and mixtures thereof. However, propofol is not included in the oily component according to the invention.

Regarding the oily component according to the invention, from the viewpoint of mitigation of angialgia and the solubility of propofol, it is preferable that the oily component includes a medium-chain fatty acid triglyceride.

According to the invention, a medium-chain fatty acid triglyceride means an oil or fat in which the average number of carbon atoms of a fatty acid chain that constitutes the triglyceride contained in the medium-chain fatty acid triglyceride is 12 or less.

The average number of carbon atoms of the fatty acid in a medium-chain fatty acid triglyceride is a weighted average of the number of carbon atoms (for example, 8 for caprylic acid, and 10 for capric acid) of a fatty acid chain that constitutes the triglyceride contained in the medium-chain fatty acid triglyceride (may be referred to as "constituent fatty acid" in the present specification), on the basis of the compositional ratio of the constituent fatty acid.

Regarding the medium-chain fatty acid triglyceride used for the invention, there are no particular limitations on the constituent fatty acid, and for example, fatty acids having from 6 to 12 carbon atoms may be used. These constituent fatty acids in the medium-chain fatty acid triglyceride may be saturated or unsaturated. Preferably, the medium-chain fatty acid triglyceride is mainly composed of a triglyceride of saturated fatty acids having from 6 to 12 carbon atoms.

Furthermore, the medium-chain fatty acid triglyceride may be a product derived from a natural plant oil, or may be a triglyceride of synthetic fatty acids. These may be used singly or in combination of two or more kinds thereof. Regarding the medium-chain fatty acid triglyceride, as long as the average number of carbon atoms of the constituent fatty acid chain is within the range described above, one kind of such a triglyceride may be used alone, or a mixture of two or more kinds of medium-chain fatty acid triglycerides having constituent fatty acid chains with different average numbers of carbon atoms may also be used. In a case in which two or more kinds of medium-chain fatty acid triglycerides are mixed, it is desirable that the overall average number of carbon atoms of the constituent fatty acids of the mixture of medium-chain fatty acid triglycerides is within the range described above.

An example of the medium-chain fatty acid triglyceride that can be used for the invention may be one that conforms to the standards of "Medium-chain fatty acid triglycerides" of "Japanese Standards of Medical Package Inserts 2003 (Yakuji Nippo, Ltd.)". Examples of commercially available products of medium-chain fatty acid triglycerides include trade name: "COCONARD™" (registered trademark) (Kao Corporation), "ODO (registered trademark)™" (Nisshin Oillio Group, Ltd.), "Myglyol™" (registered trademark (Sasol, Ltd.), and "Panasate™" (registered trademark) (NOF Corporation).

From the viewpoints of mitigating angialgia and dissolving a sufficient amount of propofol in the composition, the mass content of the medium-chain fatty acid triglyceride according to the invention is preferably from 30 w/w% to 100 w/w%, more preferably from 60 w/w% to 100 w/w%, and even more preferably from 70 w/w% to 100 w/w%, with respect to the total mass of the oily component.

It is preferable that the oily component according to the invention includes a long-chain fatty acid triglyceride, from the viewpoints of the operating experience in injectable preparations and the suppression of decomposition of propofol. The long-chain fatty acid triglyceride according to the present specification means an oil or fat in which the average number of carbon atoms of a fatty acid chain that constitutes the triglyceride contained in the long-chain fatty acid triglyceride is larger than 12. The fatty acid that constitutes the fatty acid chain may be a saturated fatty acid or an unsaturated fatty acid. Examples of the long-chain fatty acid triglyceride include plant oils corresponding to natural triglycerides, and chemically synthesized triglycerides.

Specific examples of the plant oils include soybean oil, cotton seed oil, rapeseed oil, sesame oil, safflower oil, corn oil, peanut oil, olive oil, coconut oil, perilla oil, and castor oil. Among them, from the viewpoint of the operating experience in injectable preparations, soybean oil is preferred.

Soybean oil (soya bean oil) is a plant oil obtained from seeds of plants of the genus Glycine in the family Fabaceae, and can be obtained from seeds using known extraction methods and known purification methods. For example, soybean oil that conforms to the standards of "Soybean Oil" described in the Japanese Pharmacopeia can be used. Examples of commercially available products of soybean oil include "Japanese Pharmacopeia Soybean Oil" (Kaneda Corporation), "SOYBEANOIL YM" (Nisshin Oillio Group, Ltd.), and SR-SOYBEAN-LQ-(JP) (Croda Japan K.K.).

Examples of the chemically synthesized triglycerides include 2-linoleoyl-1,3-dioctanoyl glycerol.

From the viewpoint of suppressing decomposition of propofol, the mass content of the long-chain fatty acid triglyceride is preferably 10 w/w% or more and less than 70 w/w% with respect to the total mass of the oily component. Particularly, it is preferable that the oily component is composed of two components, namely, the medium-chain fatty acid triglyceride and the long-chain fatty acid triglyceride, and in this case, it is particularly preferable that the mass content of the long-chain fatty acid triglyceride is less than 40 w/w%, and particularly preferably more than 20 w/w% and less than 30 w/w%, of the total mass of the oily component.

According to the invention, these oily components can be utilized singly or in combination of two or more kinds thereof. In a case in which two or more kinds of oily components are used in combination, it is not necessary that the various components to be used in combination be selected from the same group of compounds, such as plant oils, medium-chain fatty acid triglycerides, animal oils, and mineral oils, and can be selected from different groups.

The emulsion composition of the invention includes an emulsifier in order to constitute an oil-in-water emulsion composition containing propofol.

Regarding the emulsifier, phospholipids can be used, and for example, lecithins, which are naturally occurring phospholipids, may be used. Examples of the lecithins include egg yolk lecithin, egg yolk phosphatidylcholine, soybean lecithin, soybean phosphatidylcholine; and hydrogenated egg yolk lecithin, hydrogenated egg yolk phosphtidylcholine, hydrogenated soybean lecithin, and hydrogenated soybean phosphatidylcholine, which are obtained by hydrogenating the aforementioned substances.

The phospholipids are not limited to naturally occurring components, and chemically synthesized phospholipids may also be used. Examples of the chemically synthesized phospholipids include phosphatidylcholines (dipalmitoylphosphatidylcholine, dimyristoylphosphatidylcholine, distearoylphosphatidylcholine, dioleoylphosphatidylcholine, and the like), phosphatidicylglycerols (dipalmitoylphosphatidylglycerol, dimyristoylphosphatidylglycerol, distearoylphosphatidylglycerol, dioleoylphosphatidylglycerol, and the like), and phosphatidylethanolamines (dipalmitoylphosphatidylethanolamine, dimyristoylphosphatidylethanolamine, distearoylphosphatidylethanolamine, dioleoylphosphatidylethanolamine, and the like).

These phospholipids can be utilized singly or as mixtures of two or more kinds thereof.

From the viewpoint of biocompatibility, more preferred examples of the phospholipids include egg yolk lecithin, egg yolk phosphatidylcholine, soybean lecithin, and soybean phosphatidylcholine, and egg yolk lecithin is particularly preferred.

In regard to such an emulsion composition of the invention, it is preferable that the oily component and the emulsifier are included in an amount of from 1.2 w/v% to 11.0 w/v%, as the sum total mass content, with respect to the total capacity of the composition. This sum total amount of the oily component and the emulsifier is generally an amount smaller than the above-mentioned sum total amount used in a propofol-containing oil-in-water emulsion composition. When such a sum total amount is employed, for example, the influence caused by fat load, for example, occurrence of hyperlipidemia, can be reduced. If the sum total amount of the oily component and the emulsifier is more than 11.0 w/v%, there are occasions in which influence caused by fat load, such as the occurrence of hyperlipidemia, may be expected. If the sum total amount of the oily component and the emulsifier is less than 1.2 w/v%, the emulsion composition cannot contain a sufficient amount of propofol.

The sum total mass content of the combination of the oily component and the emulsifier is more preferably from 4.0 w/v% to 11.0 w/v%, and even more preferably from 6.0 w/v% to 10.0 w/v%, with respect to the total capacity of the composition. When the sum total mass content is 1.2 w/v% or more, the emulsion composition can contain a sufficient amount of propofol, and when the sum total mass content is 11.0 w/v% or less, it is preferable because the fat load can be sufficiently reduced.

Regarding the mass content of the oily component in the emulsion composition, it is preferable that the sum total amount of the mass content of the oily component and the mass content of the emulsifier does not exceed 11.0 w/v% with respect to the total capacity of the emulsion composition, and the mass content of the oily component is preferably 1.0 to 10.0 w/v%, more preferably 1.0 to 9.0 w/v%, even more preferably 2.0 to 8.5 w/v%, and particularly preferably 3.0 to 7.0 w/v%, with respect to the total capacity of the emulsion composition. When the mass content is 1.0 w/v% or more, the emulsion composition can contain the drug at a sufficient concentration, and when the mass content is 11.0 w/v% or less, stability of the emulsion composition is not impaired, so that it is preferable in the respective cases.

Regarding the mass content of the emulsifier, it is preferable that the sum total amount of the mass content of the emulsifier and the mass content of the oily component does not exceed 11.0 w/v% with respect to the total capacity of the composition, and the mass content of the emulsifier is preferably 0.4 to 1.2 w/v%, more preferably 0.5 to 1.2 w/v%, and particularly preferably 0.6 to 1.2 w/v%, with respect to the total capacity of the emulsion composition. When the mass content is 0.4 w/v% or more, the emulsion composition is sufficiently stable, and when the mass content is 1.2 w/v% or less, this does not fall under excessive administration in view of the occurrence of fat load, and it is almost unnecessary to consider the influence caused by such excessive administration.

The ratio of the mass content of the emulsifier to the mass content of the oily component is preferably from 0.04 to 0.2, more preferably from 0.06 to 0.2, and most preferably from 0.1 to 0.2. When the mass ratio of the emulsifier with respect to the oily component is 0.04 or more, the emulsion composition is sufficiently stable, and when the mass ratio is 0.2 or less, there is a tendency that insoluble matters are not easily produced in a case in which the emulsion composition is subjected to long-term storage.

The propofol-containing oil-in-water emulsion composition of the invention may preferably include propofol at a proportion of 0.1 to 5 w/v%, the emulsifier at a proportion of 0.1 to 2 w/v%, and the oily component at a proportion of 0.1 to 9.9 w/v%.

The propofol-containing oil-in-water emulsion composition of the invention further includes at least one selected from the group consisting of trishydroxymethylaminomethane, phosphoric acid, and triethanolamine (hereinafter, may be referred to as buffering agent). The present emulsion composition can achieve suppression of decomposition of propofol by having the buffering agent incorporated therein. Regarding the buffering agent, from the viewpoints of the particle size and the suppression of decomposition of propofol, it is more preferable to use trishydroxymethylaminomethane.

According to the invention, trishydroxymethylaminomethane means trishydroxymethylaminomethane or a salt thereof. Examples of the salt of trishydroxymethylaminomethane include trishydroxymethylaminomethane hydrochloride.

In the case of using trishydroxymethylaminomethane, the mass content is preferably from 0.005 w/v% to 0.5 w/v%, and more preferably from 0.01 w/v% to 0.2 w/v%, with respect to the total capacity of the composition, from the viewpoint of suppressing decomposition of propofol.

According to the invention, phosphoric acid means phosphoric acid or a salt thereof.

In the case of using phosphoric acid, phosphoric acid can be added in the form of a phosphate (for example, disodium hydrogen phosphate or sodium dihydrogen phosphate), and the mass content of the phosphate is preferably from 0.01 w/v% to 1 w/v%, and more preferably from 0.05 w/v% to 0.5 w/v%, with respect to the total capacity of the composition, from the viewpoint of suppressing decomposition of propofol.

According to the invention, triethanolamine means triethanolamine or a salt thereof. Examples of the salt of triethanolamine include triethanolamine hydrochloride.

In the case of using triethanolamine, the mass content thereof is preferably from 0.0005 w/v% to 0.05 w/v%, and more preferably from 0.001 w/v% to 0.01 w/v%, with respect to the total capacity of the composition, from the viewpoint of suppressing decomposition of propofol.

When the mass contents of trishydroxymethylaminomethane, phosphoric acid, and triethanolamine are adjusted to be in the ranges described above, an effect of suppressing decomposition of propofol can be sufficiently obtained, and it is almost unnecessary to consider the influence of excessive administration, so that it is preferable in the respective cases. It is also preferable in view of emulsion stability.

It is preferable that the propofol-containing oil-in-water emulsion composition of the invention further includes an antioxidant, and it is preferable that the emulsion composition includes, as the antioxidant, at least one selected from the group consisting of citric acid or a salt thereof, ascorbic acid or a salt thereof, and a thiol-based antioxidant. The antioxidant is more preferably a thiol-based antioxidant. When the emulsion composition further includes at least one selected from the group consisting of citric acid or a salt thereof, ascorbic acid or a salt thereof, and a thiol-based antioxidant (hereinafter, these may be collectively referred to as antioxidants), stability of propofol can be further enhanced. It is described in JP2005-538191A that ascorbic acid decomposes propofol. However, in the invention, in contrast to the finding of JP2005-538191A, it was demonstrated that decomposition of propofol can be suppressed by adding an appropriate amount of ascorbic acid to the emulsion composition.

The salt of citric acid and the salt of ascorbic acid may be any pharmacologically acceptable salts, and examples include salts of citric acid or ascorbic acid and alkali metals; salts of citric acid or ascorbic acid and alkaline earth metals; salts of citric acid or ascorbic acid and transition metals; and salts of citric acid or ascorbic acid and basic ammonium.

Specific examples include alkali metal salts with sodium, potassium, and the like; alkaline earth metal salts with calcium, magnesium, and the like; transition metal salts with zinc, iron, cobalt, copper, and the like; and basic ammonium salts with ammonium, triethanolamine, L-histidine, L-arginine, L-lysine, and the like.

Among them, examples of citric acid or ascorbic acid include trisodium citrate, disodium citrate, sodium dihydrogen citrate, disodium hydrogen citrate, tripotassium citrate, dipotassium citrate, potassium dihydrogen citrate, dipotassium hydrogen citrate, sodium ascorbate, potassium ascorbate, magnesium ascorbate, calcium ascorbate, sodium ascorbate phosphate, and magnesium ascorbate phosphate. From the viewpoint of suppressing decomposition of propofol, it is preferable to use trisodium citrate or ascorbic acid; however, there are no particular limitations.

The thiol-based antioxidant is not particularly limited as long as it is a compound having a thiol group, and examples thereof include thioglycerol, cysteine or a salt thereof, thioglycolic acid or a salt thereof, thiomalic acid or a salt thereof, glutathione, a polypeptide containing cysteine residues, methylmercaptan, aminoethylthiol, N-acetyl-L-cysteine, 2-mercaptoethanol, and dithioerythritol. The thiol-based antioxidant is preferably cysteine or a salt thereof, thioglycolic acid or a salt thereof, or thioglycerol, from the viewpoint of suppressing decomposition of propofol. The salts of cysteine and thioglycolic acid may be any pharmacologically acceptable salts, and examples include salts of cysteine or thioglycolic acid with alkali metals; salts of cysteine or thioglycolic acid with alkaline earth metals; salts of cysteine or thioglycolic acid with transition metals; and salts of cysteine or thioglycolic acid with basic ammonium.

Specific examples include alkali metal salts with sodium, potassium, and the like; alkaline earth metal salts with calcium, magnesium, and the like; transition metal salts with zinc, iron, cobalt, copper, and the like; basic ammonium salts with ammonium, triethanolamine, L-histidine, L-arginine, L-lysine, and the like; hydrochlorides, formates, acetates, maleates, fumarates, and tartrates.

Among them, examples of the salts of cysteine include cysteine hydrochloride, and examples of the salts of thioglycolic acid include sodium thioglycolate and potassium thioglycolate.

Regarding the antioxidant, the case in which citric acid or a salt thereof and a thiol-based antioxidant are incorporated in combination, and the case in which ascorbic acid or a salt thereof, and a thiol-based antioxidant are incorporated in combination are preferred.

In the case of using citric acid or a salt thereof, the mass content of citric acid or a salt thereof is preferably from 1 w/v% to 40 w/v%, and more preferably from 2 w/v% to 10 w/v%, in terms of trisodium citrate dihydrate with respect to propofol, from the viewpoint of suppressing decomposition of propofol. When the mass content is 1 w/v% or more, an effect of suppressing decomposition of propofol provided by citric acid or a salt thereof can be sufficiently obtained, and when the mass content is 40 w/v% or less, it is almost unnecessary to consider the influence of excessive administration, so that it is preferable in the respective cases. Furthermore, from the viewpoint of emulsion stability, the mass content is preferably 10 w/v% or less.

In the case of using ascorbic acid or a salt thereof, the mass content of ascorbic acid or a salt thereof is preferably from 0.1 w/v% to 60 w/v%, and more preferably from 0.5 w/v% to 10 w/v%, in terms of ascorbic acid with respect to propofol, from the viewpoint of suppressing decomposition of propofol. When the mass content is 0.1 w/v% or more, an effect of suppressing decomposition of propofol provided by ascorbic acid or a salt thereof can be sufficiently obtained, and when the mass content is 60 w/v% or less, it is almost unnecessary to consider the influence of excessive administration, so that it is preferable in the respective cases. Furthermore, from the viewpoint of emulsion stability, the mass content is preferably 10 w/v% or less.

In the case of using cysteine or a salt thereof, the mass content of cysteine or a salt thereof is preferably from 0.01 w/v% to 5 w/v%, and more preferably from 0.1 w/v% to 4 w/v%, in terms of cysteine hydrochloride with respect to propofol, from the viewpoint of suppressing decomposition of propofol. When the mass content is 0.01 w/v% or more, an effect of suppressing decomposition of propofol provided by cysteine or a salt thereof can be sufficiently obtained, and when the mass content is 5 w/v% or less, it is almost unnecessary to consider the influence of excessive administration, so that it is preferable in the respective cases.

In the case of using thioglycolic acid or a salt thereof, the mass content of thioglycolic acid or a salt thereof is preferably from 0.01 w/v% to 0.5 w/v%, and more preferably from 0.1 w/v% to 0.4 w/v%, in terms of sodium thioglycolate with respect to propofol, from the viewpoint of suppressing decomposition of propofol. When the mass content is 0.01 w/v% or more, an effect of suppressing decomposition of propofol provided by thioglycolic acid or a salt thereof can be sufficiently obtained, and when the mass content is 0.5 w/v% or less, it is almost unnecessary to consider the influence of excessive administration, so that it is preferable in the respective cases. It is also preferable from the viewpoint of emulsion stability.

In the case of using thioglycerol, the mass content of thioglycerol is preferably from 0.01 w/v% to 1 w/v%, and more preferably from 0.1 w/v% to 0.8 w/v%, in terms of thioglycerol with respect to propofol, from the viewpoint of suppressing decomposition of propofol. When the mass content is 0.01 w/v% or more, an effect of suppressing decomposition of propofol provided by thioglycerol can be sufficiently obtained, and when the mass content is 1 w/v% or less, it is almost unnecessary to consider the influence of excessive administration, so that it is preferable in the respective cases.

For the emulsion composition of the invention, a combination including trishydroxymethylaminomethane and a thiol-based antioxidant as an antioxidant is preferred. Furthermore, the case in which trishydroxymethylaminomethane, and a thiol-based antioxidant and citric acid or a salt thereof as antioxidants are included in combination, and the case in which trishydroxymethylaminomethane, and a thiol-based antioxidant and ascorbic acid or a salt thereof as antioxidants are included in combination are more preferred. In this case, the case in which two or more kinds of thiol-based antioxidants selected from the group consisting of cysteine or a salt thereof, thioglycolic acid or a salt thereof, and thioglycerol are used in combination as the thiol-based antioxidant is more preferred.

In regard to the emulsion composition of the invention, although not particularly required, if desired, adequate amounts of various additives that are known to be possibly added and blended into this kind of emulsion composition can be further added and blended into the composition. Examples of the additives include an antibacterial agent, a pH adjusting agent, and an isotonic agent.

Examples of the antibacterial agent include sodium caprylate, methyl benzoate, and sodium metabisulfite.

Examples of the pH adjusting agent that can be used include hydrochloric acid, acetic acid, lactic acid, malic acid, and sodium hydroxide.

Examples of the isotonic agent include glycerin; sugars such as glucose, fructose, and maltose; sugar alcohols such as sorbitol and xylytol; and salts such as sodium chloride and magnesium chloride.

Among these, oil-soluble substances can be utilized after being mixed in advance with the oily component that constitutes the emulsion composition. Water-soluble substances can be mixed into the water for injection, or can be added and blended into the aqueous phase of the emulsion liquid thus obtainable. The amounts of addition and blending of these substances are not especially different from those amounts of addition and blending that are obvious and conventionally known to those ordinarily skilled in the art.

The absolute value of the zeta potential of the propofol-containing oil-in-water emulsion composition of the invention is not particularly limited; however, the absolute value is preferably 30 mV or less, and more preferably 20 mV or less.

The zeta potential is generally measured by a method combining electrophoresis and light scattering, for example, an electrophoretic light scattering measurement method (laser Doppler method). The present method involves applying an electric field to particles to move the particles (electrophoresis), irradiating the moving particles with laser light, determining the migration speed of the particles from the changes in the frequencies of radiated light and scattered light, and thereby calculating the zeta potential. The zeta potential can be measured using, for example, NANO-ZS manufactured by Malvern Instruments, Ltd., and zeta potential analyzer ELS-8000 manufactured by Otsuka Electronics Co., Ltd.

The pH of the propofol-containing oil-in-water emulsion composition of the invention is not particularly limited; however, the pH is usually 5.0 to 9.0, and preferably 6.0 to 8.5.

The propofol-containing oil-in-water emulsion composition of the invention can be produced according to a method known in the pertinent art (emulsification dispersion method).

Preferably, the propofol-containing oil-in-water emulsion composition of the invention can be produced by a process of treating a composition including propofol, an oily component, an emulsifier, and water using a high pressure homogenizer under the conditions of a pressure of 200 MPa or higher; and a process of sterilizing the composition thus obtained by passing the composition through a filter having a pore size of 0.22 μm or less.

For example, a method of mixing an aqueous phase and oil phase to roughly emulsify the phases, and then emulsifying the resulting rough emulsion by utilizing an appropriate high pressure emulsifying machine or the like (fine emulsification) can be employed. More specifically, rough emulsification can be performed using, for example, a HOMOMIXER such as T.K. HOMOMIXER manufactured by Primix Corporation, usually requiring 5 minutes or longer at a rate of 5,000 rotations/min or more. Furthermore, an ultrasonic homogenizer can also be used. Fine emulsification can be performed using a high pressure homogenizer, an ultrasonic homogenizer, or the like. In the case of using a high pressure homogenizer, fine emulsification can be generally performed by passing the emulsion through the homogenizer about 1 to 50 times, and preferably about 1 to 20 times, under the conditions of a pressure of about 150 MPa or higher. Such a mixing and emulsification operation may be carried out at normal temperature, or may be carried out by employing a light cooling operation or a warming operation.

The emulsion composition of the invention can be produced into a manufactured product by adjusting the pH as necessary, and then performing sterilization by filtration according to a conventional method. Regarding the method of sterilization by filtration, for example, a known method of using a filter may be applied. The material for the filter is not particularly limited; however, the material is preferably cellulose acetate. A general sterilization method may be, for example, high pressure steam sterilization (for example, 121°C, 20 minutes) is available; however, according to the invention, decomposition of propofol caused by high pressure steam sterilization can be avoided by making the particle size small, and enabling sterilization by filtration.

Regarding the method for adding a buffering agent, at least one selected from the group consisting of trishydroxymethylaminomethane, phosphoric acid, and triethanolamine may be added to the composition including propofol before rough emulsification, may be added after rough emulsification, or may be added to the composition after fine emulsification, or the buffering agent may be sterilized in a state of being dissolved in water and added to the composition after sterilization using a filter. Also, the buffering agent may be added at a plurality of time points, instead of being added once.

Addition of the antioxidant is also similar to the addition of the buffering agent.

The propofol-containing oil-in-water emulsion composition of the invention is an emulsion composition in which decomposition of propofol is suppressed.

The effect of suppressing decomposition of propofol can be evaluated by, for example, detection of a propofol dimer. Detection of the propofol dimer can be performed by employing a known method, and for example, the propofol dimer can be detected by measurement by high performance liquid chromatography (HPLC).

In the following description, the invention will be explained more specifically by way of Examples of the invention. The materials, amounts of use, proportions, treatments, procedures, and the like described in the following Examples can be appropriately modified as long as the gist of the invention is maintained. Therefore, the scope of the invention is not to be construed to be limited to the specific examples disclosed below.

EXAMPLES

(Raw materials used in Examples)

Propofol: "2,6-Diisopropylphenol" (manufactured by Bachem AG)
Japanese Pharmacopeia soybean oil: "Japanese Pharmacopeia Soybean Oil" (manufactured by Kaneda Corporation)
Medium-chain fatty acid triglyceride: "MYGLYOL 812" (manufactured by Sasol, Ltd.)
Purified egg yolk lecithin: "Egg Yolk Lecithin PL100-M" (manufactured by Qatar Petroleum Company)
Japanese Pharmacopeia concentrated glycerin: "Japanese Pharmacopeia Concentrated Glycerin" (manufactured by Sakamoto Yakuhin Kogyo Co., Ltd.)
Trishydroxymethylaminomethane: "for production of TROMETAMOL" (manufactured by Wako Pure Chemical Industries, Ltd.)
Triethanolamine: "Triethanolamine" (manufactured by Tokyo Chemical Industry Co., Ltd.)
Disodium hydrogen phosphate: "Disodium Hydrogen Phosphate" (manufactured by Wako Pure Chemical Industries, Ltd.)
Sodium dihydrogen phosphate: "Sodium Dihydrogen Phosphate" (manufactured by Wako Pure Chemical Industries, Ltd.)
Sodium citrate: "Trisodium Citrate Dihydrate" (manufactured by Wako Pure Chemical Industries, Ltd.)
Ascorbic acid: "Ascorbic acid" (manufactured by DSM Nutrition Japan K.K.)
Cysteine hydrochloride: "L-Cysteine Hydrochloride Hydrate" (manufactured by Kyowa Hakko Bio Co., Ltd.)
Sodium thioglycolate: "Sodium Thioglycolate" (manufactured by Tokyo Chemical Industry Co., Ltd.)

Sodium hydrogen sulfite: "Sodium Hydrogen Sulfite" (manufactured by Wako Pure Chemical Industries, Ltd.)
Sodium sulfite: "Sodium Sulfite" (manufactured by Wako Pure Chemical Industries, Ltd.)
Sodium pyrosulfite: "Sodium Disulfite" (manufactured by Wako Pure Chemical Industries, Ltd.)
Alpha-thioglycerol: "1-Thioglycerol" (manufactured by Asahi Kagaku Kogyo Co., Ltd.)
Sodium oleate: "Sodium Oleate" (manufactured by Wako Pure Chemical Industries, Ltd.)
1 mol/L Hydrochloric acid (manufactured by Wako Pure Chemical Industries, Ltd.)
1 mol/L Sodium hydroxide (manufactured by Wako Pure Chemical Industries, Ltd.)

[Examples 1 to 14, Reference Example 1, and Comparative Examples 1 and 2]

(Production of propofol-containing emulsion composition)

Propofol-containing oil-in-water emulsion compositions were produced by the following procedure, using the various components described in Table 1 at the contents indicated in the table (the numbers in the table represent w/v% with respect to the total capacity of the composition).

Various medium-chain fatty acid triglycerides were mixed with soybean oil, propofol was added thereto, and the mixture was stirred and dissolved. Thus, an oil phase was produced. Glycerin was dissolved in water, and an aqueous phase was produced. Purified egg yolk lecithin was added to the oil phase and mixed, and then the aqueous phase was added thereto. The resultant was subjected to rough emulsification using a HOMOMIXER (15,000 rpm (rotations/min), 5 minutes). This was passed 5 times through a high pressure emulsifying machine (STARBURST MINILABO, Sugino Corporation) under the conditions of 245 MPa. Subsequently, additives were added thereto, and the pH was adjusted to 8.0 using 1 mol/L hydrochloric acid or 1 mol/L sodium hydroxide. Thus, an emulsion composition was produced.

In Examples 1 to 14 and Comparative Example 2, the emulsion composition thus produced was subjected to sterilization by filtration by passing the emulsion composition through SARTOBRAN P (Sartorius Stedim Japan K.K.) under a nitrogen pressure of 0.2 MPa. The emulsion composition after sterilization was stirred for 3 hours in a nitrogen atmosphere (oxygen concentration in the gas: less than 0.1 v/v%), and an INLAB 605 dissolved oxygen sensor connected to SEVEN GOPRO (manufactured by Mettler Toledo International, Inc.) was brought into contact with the liquid to thereby confirm that the dissolved oxygen concentration was 0.1 mg/L or less. Subsequently, the emulsion composition was charged into a 1-ml CZ Syringe (manufactured by Daikyo Seiko, Ltd.) that had been subjected to high pressure steam sterilization for 20 minutes at 121°C in an autoclave (AUTOCLAVE SP200, Yamato Scientific Co., Ltd.). After charging, the syringe was enclosed into LAMIZIP (manufactured by PET/AL) in a nitrogen atmosphere, and was subjected to heat sealing.

In Reference Example 1, the emulsion composition was stirred for 3 hours in a nitrogen atmosphere, and an INLAB 605 dissolved oxygen sensor connected to SEVEN GOPRO was brought into contact with the liquid to confirm that the dissolved oxygen concentration was 0.1 mg/L or less. Subsequently, the emulsion composition was charged into a "VIAL WHITE V-NT, 10 mL CS SILICOAT" (manufactured by Fuji Glass Co., Ltd.) that had been subjected to high pressure steam sterilization for 20 minutes at 121°C using an autoclave. Each charged vial was subjected to high pressure steam sterilization for 20 minutes at 121°C using an autoclave.

In Comparative Example 1, the emulsion composition thus produced was charged into a 1-ml CZ SYRINGE that had been subjected to high pressure steam sterilization for 20 minutes at 121°C using an autoclave, and then the whole syringe was subjected to high pressure steam sterilization for 20 minutes at 121°C using an autoclave.

[Table 1-1]

|  |  | Example 1 | Example 2 | Example 3 | Example 4 | Example 5 | Example 6 | Example 7 |
|---|---|---|---|---|---|---|---|---|
| Oil phase | Propofol | 1 | 1 | 1 | 1 | 1 | 1 | 1 |
|  | MYGLYOL 812 | 5 | 5 | 5 | 5 | 5 | 5 | 5 |
|  | Japanese Pharmacopeia soybean oil | 1.5 | 1.5 | 1.5 | 1.5 | 1.5 | 1.5 | 1.5 |
| Aqueous phase | Japanese pharmacopeia glycerin | 2.25 | 2.25 | 2.25 | 2.25 | 2.25 | 2.25 | 2.25 |
|  | Water | Appropriate | Appropriate | Appropriate | Appropriate | Appropriate | Appropriate | Appropriate |

| | | amount | amount | amount | amount | amount | amount | amount |
|---|---|---|---|---|---|---|---|---|
| Purified egg yolk lecithin | | 1.2 | 1.2 | 1.2 | 1.2 | 1.2 | 1.2 | 1.2 |
| Additives | Trishydroxymethyl aminomethane | 0.053 | - | - | 0.053 | 0.053 | 0.053 | 0.053 |
| | Disodium hydrogen phosphate | - | 0.163 | - | - | - | - | - |
| | Sodium dihydrogen phosphate | - | 0.0016 | - | - | - | - | - |
| | Triethanolamine | - | - | 0.006 | - | - | - | - |
| | Sodium citrate | - | - | - | 0.0882 | - | - | - |
| | Cysteine hydrochloride | - | - | - | - | 0.0034 | - | - |
| | Sodium thioglycolate | - | - | - | - | - | 0.004 | - |
| | Ascorbic acid | - | - | - | - | - | - | 0.01 |
| | Alpha-thioglycerol | - | - | - | - | - | - | - |

| | | Example 8 | Example 9 | Example 10 | Example 11 | Example 12 | Example 13 | Example 14 |
|---|---|---|---|---|---|---|---|---|
| Oil phase | Propofol | 1 | 1 | 1 | 1 | 1 | 1 | 1 |
| | MYGLYOL 812 | 5 | 5 | 5 | 5 | 5 | 5 | 5 |
| | Japanese Pharmacopeia soybean oil | 1.5 | 1.5 | 1.5 | 1.5 | 5 | 5 | 1.5 |
| Aqueous phase | Japanese pharmacopeia glycerin | 2.25 | 2.25 | 2.25 | 2.25 | 2.25 | 2.25 | 2.25 |
| | Water | Appropriate amount | Appropriate amount | Appropriate amount | Appropriate amount | Appropriate amount | Appropriate amount | Appropriate amount |
| Purified egg yolk lecithin | | 1.2 | 1.2 | 1.2 | 1.2 | 1.2 | 1.2 | 1.2 |
| Additives | Trishydroxymethyl aminomethane | 0.053 | 0.053 | 0.053 | 0.053 | 0.053 | 0.053 | 0.053 |
| | Disodium hydrogen phosphate | - | - | - | - | - | - | - |
| | Sodium dihydrogen phosphate | - | - | - | - | - | - | - |
| | Triethanolamine | - | - | - | - | - | - | - |
| | Sodium citrate | - | 0.0441 | 0.0441 | 0.0882 | 0.0441 | - | 0.0822 |
| | Cysteine hydrochloride | - | 0.02 | 0.0034 | 0.0034 | 0.0034 | 0.0034 | 0.0034 |
| | Sodium thioglycolate | - | - | - | 0.004 | 0.004 | 0.004 | 0.004 |
| | Ascorbic acid | - | - | - | - | - | 0.01 | 0.01 |
| | Alpha-thioglycerol | 0.005 | - | 0.005 | - | - | - | - |

[Table 1-2]

| | | Reference Example 1 | Comparative Example 1 | Comparative Example 2 |
|---|---|---|---|---|
| Oil phase | Propofol | 1 | 1 | 1 |
| | MYGLYOL 812 | 5 | 5 | 5 |
| | Japanese Pharmacopeia soybean oil | 5 | 5 | 1.5 |

(continued)

| | | Reference Example 1 | Comparative Example 1 | Comparative Example 2 |
|---|---|---|---|---|
| Aqueous phase | Japanese pharmacopeia glycerin | 2.25 | 2.25 | 2.25 |
| | Water | Appropriate amount | Appropriate amount | Appropriate amount |
| Purified egg yolk lecithin | | 1.2 | 1.2 | 1.2 |
| Additives | Trishydroxymethylaminomethane | - | - | - |
| | Disodium hydrogen phosphate | - | - | - |
| | Sodium dihydrogen phosphate | - | - | - |
| | Triethanolamine | - | - | - |
| | Sodium citrate | - | - | - |
| | Cysteine hydrochloride | - | - | - |
| | Sodium thioglycolate | - | - | - |
| | Ascorbic acid | - | - | - |
| | Alpha-thioglycerol | - | - | - |

(Thermal Severe Test I)

In Examples 1 to 14 and Comparative Example 2, an emulsion composition was stored in a constant-temperature tank at 60°C for one week in a state of being enclosed in LAMIZIP, and thus a thermal severe test was performed.

(Thermal Severe Test II)

In Example 4 to Example 14 and Comparative Example 2, an emulsion composition was stored in a constant-temperature tank at 60°C for 48 days in a state of being enclosed in LAMIZIP, and thus a thermal severe test was performed.

(Evaluation of stability)

400 mg of each of the emulsion compositions produced in Examples and Comparative Examples as described in Table 1 was taken and weighed before storage and after Thermal Severe Test I and Thermal Severe Test II, and the emulsion composition was diluted to 5 ml with an eluent. A dissolved sample was collected, and measurement of the concentrations of propofol and a dimer (3,3'-5,5'-tetraisopropyldiphenyl), which is an analog of propofol, was performed by high performance liquid chromatography [column: CADENZA CD-C 18 (Imtakt Corporation), eluent: 40 mass% aqueous solution of tetrahydrofuran, flow rate: 0.8 mL/min, column temperature: 40°C, detector: ultraviolet (UV) detector, detection wavelength: 270 nm, injection amount: 10 μL]. The mass ratio of the dimer with respect to propofol was calculated, and the mass ratio as the dimer amount (%) was employed as an index of the evaluation of stability.

The evaluation criteria for the initial dimer amount (%) before storage are as follows.

A: Less than 0.030%
B: 0.030% or more and less than 0.085%
C: 0.085% or more

The evaluation criteria for the dimer amount (%) after Thermal Severe Test I are as follows.

S: Less than 0.030%
A: 0.030% or more and less than 0.085%
B: 0.085% or more and less than 0.140%
C: 0.140% or more

The evaluation criteria for the dimer amount (%) after Thermal Severe Test II are as follows.

S: Less than 0.080%
A: 0.080% or more and less than 0.200%
B: 0.200% or more and less than 0.500%
C: 0.500% or more

(Measurement of average particle size of emulsion)

Each of the emulsion compositions produced in Examples, Reference Example, and Comparative Examples as described in Table 1 was diluted 10 times to 100 times using purified water immediately after high pressure emulsification. The dilution thus obtained was used to measure the median diameter of a scattering intensity distribution that was obtained by Contin method using a concentrated system particle size analyzer FPAR-1000 (Otsuka Electronics Co., Ltd.), and the median diameter was recorded as the particle size.

The evaluation criteria for the average particle size (nm) after Thermal Severe Test I are as follows.

A: Less than 180 nm
B: 180 nm or more and less than 300 nm
C: 300 nm or more

The evaluation criteria for the average particle size (nm) after Thermal Severe Test II are as follows.

A: Less than 180 nm
B: 180 nm or more and less than 1,500 nm
C: 1,500 nm or more

(Evaluation of coloration)

Each of the emulsion compositions produced in Examples, Reference Example, and Comparative Examples as described in Table 1 was visually inspected, and it was determined whether the emulsion composition was colored as a result of a lapse of time under heat.

The evaluation criteria for coloration after Thermal Severe Test I are as follows.

A: When the emulsion composition samples obtained before and after Thermal Severe Test I are placed side by side, a difference in color cannot be determined.
B: When the emulsion composition samples obtained before and after Thermal Severe Test I are placed side by side, a difference in color can be determined.

The evaluation criteria for coloration after Thermal Severe Test II are as follows.

A: When the emulsion composition samples obtained before and after Thermal Severe Test II are placed side by side, a difference in color cannot be determined.
B: When the emulsion composition samples obtained before and after Thermal Severe Test II are placed side by side, a difference in color can be determined.

(Evaluation of pH decrease)

A decrease in pH was measured using each of the emulsion compositions produced in Examples, Reference Example, and Comparative Examples as described in Table 1 in an undiluted state, before storage and after Thermal Severe Test I, using a pH meter (apparatus product No.: F-73, manufactured by Horiba, Ltd., pH electrode: MICROTOUPH electrode 9618-10D). The evaluation criteria for the pH decrease, which is a value obtained by subtracting the pH value after Thermal Severe Test I from the pH value before Thermal Severe Test I, are as follows.

A: Less than 1.0
B: 1.0 or more

(Evaluation)

The results are presented in Table 2. The evaluation criteria for comprehensive judgment are as follows.

S: The evaluation rating for the dimer amount (%) after Thermal Severe Tests I and II is S, and the evaluation ratings for the other items are all A's.
A: The evaluation ratings for all of the items are A's.
B: The evaluation ratings for all of the items are S, A or B, and one or more items rated as B are included.
C: One or more items rated as C are included.

From Reference Example 1 and Comparative Example 1, it can be seen that stability is decreased when the container is changed to a plastic syringe, and the initial dimer amount has increased as a result of autoclave treatment. However, when the average particle size of emulsified particles is made smaller by changing the composition as in the case of Comparative Example 2, and the emulsion composition is sterilized by filtration, the initial dimer amount can be reduced even if the emulsion composition is contained in a plastic syringe.

In Comparative Example 2, the pH decrease is large, and it is not preferable; however, as shown in Examples 1 to 3, a decrease in pH can be suppressed by incorporating a buffering agent. Furthermore, in Comparative Example 2, the dimer amount after a lapse of time under heat is large, and it is not preferable; however, as shown in Examples 1 to 3, an increase in dimer generation caused by a lapse of time under heat can also be suppressed by incorporating a buffering agent. This is an unexpected effect. Above all, since the dimer generation suppressing effect is high, and the average particle size obtainable after a lapse of time under heat is also preferable, trishydroxymethylaminomethane is most preferable among the buffering agents.

As shown in Examples 4 to 8, an increase in dimer generation after a lapse of time under heat can be suppressed by further adding an antioxidant. When the antioxidant is used alone, from a comparison between Examples 4 to 8 and Reference Examples 6, 7, 9, 11 and 13 disclosed below, the dimer amount is markedly suppressed when the antioxidant is used in combination with Tris. Therefore, a combination of a thiol-based antioxidant and Tris is preferred. Furthermore, when ascorbic acid was used, coloration was observed; however, a dimer generation suppressing effect could be confirmed. This is another unexpected result that is different from the descriptions of JP2005-538191A.

From a comparison between Example 4 and Examples 9 to 12, dimer amount can be suppressed by using a plurality of antioxidants in combination. Furthermore, in Examples 9 to 12, despite the high dimer generation suppressing effect, there is no increase in the particle size compared to Example 4. Therefore, a combined use of antioxidants is preferable. Regarding the combination of these antioxidants, preferable results are obtained in all of the dimer generation suppressing effect, the particle size after a lapse of time under heat, and coloration, when a combination of sodium citrate and cysteine hydrochloride; a combination of sodium citrate, cysteine hydrochloride and sodium thioglycolate; a combination of ascorbic acid, cysteine hydrochloride and sodium thioglycolate; and a combination of sodium citrate, cysteine hydrochloride and alpha-thioglycerol are employed. When four types of sodium citrate, ascorbic acid, cysteine hydrochloride, and sodium thioglycolate are all used together, the combination is not preferable compared to the combinations described above, from the viewpoint of the particle size after a lapse of time under heat.

Among these antioxidants used, preferred ones were selected based on the results of Reference Examples 2 to 13 disclosed below.

[Table 2]

| | Initial dimer amount (%) | Dimer amount (%) after Thermal Severe Test I | Dimer amount (%) after Thermal Severe Test II | Initial average particle size (nm) | Average particle size (nm) after Thermal Severe Test I | Average particle size (nm) after Thermal Severe Test II |
|---|---|---|---|---|---|---|
| Example 1 | A(0.027) | B(0.099) | - | A(138.1) | A(139.6) | - |
| Example 2 | A(0.019) | A(0.067) | - | A(121.5) | B(283.6) | - |
| Example 3 | A(0.021) | B(0.105) | - | A(131.6) | A(136.1) | - |
| Example 4 | - | A(0.031) | A(0.114) | A(119.5) | A(120.3) | B(545.6) |
| Example 5 | - | A(0.057) | B(0.221) | A(136.5) | A(129.4) | A(137.9) |
| Example 6 | - | A(0.067) | B(0.371) | A(137.6) | A(136.9) | A(131.5) |
| Example 7 | - | S(0.020) | A(0.083) | A(130.0) | A(134.9) | A(128.6) |
| Example 8 | - | B(0.088) | B(0.481) | A(136.5) | A(138.2) | A(129.5) |
| Example 9 | - | S(0.026) | A(0.076) | A(147.4) | A(146.1) | A(154.3) |
| Example 10 | - | S(0.027) | S(0.080) | A(151.0) | A(149.7) | A(168.4) |
| Example 11 | - | S(0.019) | S(0.062) | A(131.0) | A(132.7) | B(513.0) |
| Example 12 | - | S(0.020) | S(0.073) | A(128.4) | A(133.3) | A(138.4) |
| Example 13 | - | S(0.020) | S(0.062) | A(134.5) | A(135.7) | A(165.4) |
| Example 14 | - | S(0.018) | S(0.022) | A(134.4) | B(265.7) | B(1039.9) |
| Reference Example 1 | A(0.027) | - | - | - | - | - |
| Comparative Example 1 | C(0.103) | - | - | - | - | - |
| Comparative Example 2 | A(0.027) | C(0.142) | C(0.761) | A(138.1) | A(130.2) | A(142.2) |

| | Coloration | Initial pH | pH after Thermal Severe Test I | pH decrease | Evaluation of comprehensive judgment |
|---|---|---|---|---|---|
| Example 1 | - | 7.7 | 7.4 | A(0.3) | B |
| Example 2 | - | 7.6 | 7.3 | A(0.3) | B |
| Example 3 | - | 7.0 | 6.7 | A(0.3) | B |
| Example 4 | A | 7.9 | 7.7 | A(0.2) | B |
| Example 5 | A | 7.9 | 7.9 | A(0) | B |
| Example 6 | A | 8.1 | 7.9 | A(0.2) | B |
| Example 7 | B | 7.3 | 7.1 | A(0.2) | B |
| Example 8 | A | 7.9 | 7.7 | A(0.2) | B |
| Example 9 | A | 8.1 | 8.1 | A(0) | S |
| Example 10 | A | 8.1 | 7.9 | A(0.2) | S |
| Example 11 | A | 8.2 | 8.0 | A(0.2) | B |
| Example 12 | A | 8.3 | 8.0 | A(0.3) | S |
| Example 13 | A | 8.1 | 7.9 | A(0.2) | S |
| Example 14 | A | 8.2 | 8.1 | A(0.1) | B |
| Reference Example 1 | - | - | - | - | - |
| Comparative Example 1 | - | - | - | - | - |
| Comparative Example 2 | C | 8.3 | 6.7 | B(1.6) | C |

16

[Reference Examples 2 to 13]

(Production of propofol-containing emulsion composition)

Propofol-containing oil-in-water emulsion compositions were produced by the following procedure, using the various components described in Table 3 at the contents described in the table (the numbers in the table represent w/v% with respect to the total capacity of the composition).

Various medium-chain fatty acid triglycerides were mixed with soybean oil, propofol was added thereto, and the mixture was stirred and dissolved. Thus, an oil phase was produced. Glycerin was dissolved in water, and an aqueous phase was produced. Purified egg yolk lecithin was added to the oil phase and mixed, and then the aqueous phase was added thereto. The resultant was subjected to rough emulsification using a HOMOMIXER (15,000 rpm (rotations/min), 5 minutes). This was passed 5 times through a high pressure emulsifying machine (STARBURST MINILABO, Sugino Corporation) under the conditions of 245 MPa. Subsequently, additives were added thereto, and thus, an emulsion composition was produced.

The emulsion composition thus produced was subjected to sterilization by filtration similarly to Examples 1 to 14 and Comparative Example 2, and was stirred for 3 hours in a nitrogen atmosphere. It was confirmed that the dissolved oxygen concentration was 0.1 mg/L or less (measurement temperature: room temperature, pressure: atmospheric pressure), subsequently the emulsion composition was charged into a 1-ml CZ SYRINGE (manufactured by Daikyo Seiko, Ltd.) that had been subjected to high pressure steam sterilization, and the syringe was enclosed in LAMIZIP.

[Table 3]

| | | Reference Example 2 | Reference Example 3 | Reference Example 4 | Reference Example 5 | Reference Example 6 | Reference Example 7 |
|---|---|---|---|---|---|---|---|
| Oil phase | Propofol | 1 | 1 | 1 | 1 | 1 | 1 |
| | MYGLYOL 812 | 5 | 5 | 5 | 5 | 5 | 5 |
| | Japanese Pharmacopeia soybean oil | 1.5 | 1.5 | 1.5 | 1.5 | 1.5 | 1.5 |
| Aqueous phase | Japanese pharmacopeia glycerin | 2.25 | 2.25 | 2.25 | 2.25 | 2.25 | 2.25 |
| | Water | Appropriate amount | Appropriate amount | Appropriate amount | Appropriate amount | Appropriate amount | Appropriate amount |
| Purified egg yolk lecithin | | 1.2 | 1.2 | 1.2 | 1.2 | 1.2 | 1.2 |
| Additives | Sodium hydrogen sulfite | 0.25 | - | - | - | - | - |
| | Sodium sulfite | - | 0.012 | - | - | - | - |
| | Sodium pyrosulfite | - | - | 0.012 | - | - | - |
| | Sodium citrate | - | - | - | 0.3528 | 0.0882 | - |

| | | | | | | |
|---|---|---|---|---|---|---|
| Cysteine hydrochloride | - | - | - | - | - | 0.0034 |
| Sodium thioglycolate | - | - | - | - | - | - |
| Alpha-thioglycerol | - | - | - | - | - | - |
| Ascorbic acid | - | - | - | - | - | - |

| | | Reference Example 8 | Reference Example 9 | Reference Example 10 | Reference Example 11 | Reference Example 12 | Reference Example 13 |
|---|---|---|---|---|---|---|---|
| Oil phase | Propofol | 1 | 1 | 1 | 1 | 1 | 1 |
| | MYGLYOL 812 | 5 | 5 | 5 | 5 | 5 | 5 |
| | Japanese Pharmacopeia soybean oil | 1.5 | 1.5 | 1.5 | 1.5 | 1.5 | 1.5 |
| Aqueous phase | Japanese pharmacopeia glycerin | 2.25 | 2.25 | 2.25 | 2.25 | 2.25 | 2.25 |
| | Water | Appropriate amount | Appropriate amount | Appropriate amount | Appropriate amount | Appropriate amount | Appropriate amount |
| Purified egg yolk lecithin | | 1.2 | 1.2 | 1.2 | 1.2 | 1.2 | 1.2 |
| Additives | Sodium hydrogen sulfite | - | - | - | - | - | - |
| | Sodium sulfite | - | - | - | - | - | - |
| | Sodium pyrosulfite | - | - | - | - | - | - |
| | Sodium citrate | - | - | - | - | - | - |
| | Cysteine hydrochloride | 0.00085 | - | - | - | - | - |
| | Sodium thioglycolate | - | 0.004 | 0.001 | - | - | - |
| | Alpha-thioglycerol | - | - | - | 0.005 | - | - |
| | Ascorbic acid | - | - | - | - | 0.14 | 0.01 |

(Evaluation)

Thermal Severe Test I was performed in the same manner as in Examples 1 to 14 and Comparative Example 2, and evaluation of stability, measurement of average emulsion particle size, evaluation of coloration, and pH measurement were performed.

The results are presented in Table 4. The evaluation criteria for comprehensive judgment are as follows.

S: The evaluation rating for the dimer amount (%) after Thermal Severe Test I is S, and the evaluation ratings for the other items are all A's.
A: The evaluation ratings for all of the items are A's.
B: The evaluation ratings for all of the items are S, A or B, and one or more items rated as B are included.
C: One or more items rated as C are included.

Among the antioxidants used, a dimer amount suppressing effect after a lapse of time under heat was confirmed in sodium citrate, ascorbic acid, cysteine hydrochloride, sodium thioglycolate, and alpha-thioglycerol. In regard to sodium citrate and ascorbic acid, if the amount of addition as too large, the particle size after a lapse of time under heat was not in a preferable range. Therefore, the respective amounts of addition were suppressed, and the concentrations that resulted a dimer amount after a lapse of time under heat and a particle size in preferred ranges, were found. Also, in a case in which only antioxidants are added, an oil-in-water emulsion containing propofol undergoes a decrease in pH over time, and therefore, the emulsion is not preferable as an injectable preparation.

[Table 4]

| | Dimer amount (%) after Thermal Severe Test I | Average particle size (nm) after Thermal Severe Test I | Coloration | Initial pH | pH after Thermal Severe Test I | pH decrease | Comprehensive judgment |
|---|---|---|---|---|---|---|---|
| Reference Example 2 | C(0.187) | C(1028.1) | B | 8.1 | 6.6 | B(1.5) | C |
| Reference Example 3 | C(0.161) | A(130.6) | B | 8.2 | 6.9 | B(1.3) | C |
| Reference Example 4 | C(0.342) | A(128.8) | B | 7.0 | 5.3 | B(1.7) | C |
| Reference Example 5 | A(0.044) | B(351.0) | B | 8.0 | 6.9 | B(1.1) | B |
| Reference Example 6 | A(0.047) | A(130.5) | A | 8.2 | 6.9 | B(1.3) | B |
| Reference Example 7 | B(0.096) | A(135.0) | A | 7.8 | 6.7 | B(1.1) | B |
| Reference Example 8 | B(0.091) | A(132.3) | A | 8.3 | 6.7 | B(1.6) | B |
| Reference Example 9 | B(0.112) | A(133.1) | A | 8.5 | 6.9 | B(1.6) | B |
| Reference Example 10 | B(0.113) | A(129.1) | A | 8.4 | 6.9 | B(1.5) | B |
| Reference Example 11 | B(0.129) | A(137.4) | A | 8.3 | 6.5 | B(1.8) | B |
| Reference Example 12 | S(0.019) | C(806.4) | B | 7.9 | 6.6 | B(1.3) | C |
| Reference Example 13 | S(0.021) | A(132.8) | B | 8.1 | 6.6 | B(1.5) | B |

[Examples 15 to 22 and Comparative Example 3]

(Production of propofol-containing emulsion composition)

Propofol-containing oil-in-water emulsion compositions were produced in the same manner as in Examples 1 to 14 and Comparative Example 2, using the various components described in Table 5 at the contents described in the table (the numbers in the table represent w/v% with respect to the total capacity of the composition).

Each of the emulsion compositions thus produced was subjected to sterilization by filtration in the same manner as in Examples 1 to 14 and Comparative Examples 2, and the emulsion composition was stirred in a nitrogen atmosphere. When a desired value of the dissolved oxygen concentration was reached, the emulsion composition was charged into a 1-ml CZ SYRINGE (manufactured by Daikyo Seiko, Ltd.) that had been subjected to high pressure steam sterilization, and the syringe was enclosed in LAMIZIP.

[Table 5]

| | | Example 15 | Example 16 | Example 17 | Example 18 | Example 19 |
|---|---|---|---|---|---|---|
| Oil phase | Propofol | 1 | 1 | 1 | 1 | 1 |
| | MYGLYOL 812 | 5 | 5 | 5 | 5 | 5 |
| | Japanese Pharmacopeia soybean oil | 1.5 | 1.5 | 1.5 | 1.5 | 1.5 |
| Aqueous phase | Japanese pharmacopeia glycerin | 2.25 | 2.25 | 2.25 | 2.25 | 2.25 |
| | Water | Appropriate amount | Appropriate amount | Appropriate amount | Appropriate amount | Appropriate amount |
| Purified egg yolk lecithin | | 1.2 | 1.2 | 1.2 | 1.2 | 1.2 |
| Additives | Trishydroxymethylaminomethane | 0.053 | 0.053 | 0.053 | 0.053 | 0.053 |
| Dissolved oxygen concentration (mg/L) | | 0.01 | 0.06 | 0.23 | 1.06 | 1.77 |

| | | Example 20 | Example 21 | Example 22 | Comparative Example 3 |
|---|---|---|---|---|---|
| Oil phase | Propofol | 1 | 1 | 1 | 1 |
| | MYGLYOL 812 | 5 | 5 | 5 | 5 |
| | Japanese Pharmacopeia soybean oil | 1.5 | 1.5 | 1.5 | 1.5 |
| Aqueous phase | Japanese pharmacopeia glycerin | 2.25 | 2.25 | 2.25 | 2.25 |
| | Water | Appropriate amount | Appropriate amount | Appropriate amount | Appropriate amount |
| Purified egg yolk lecithin | | 1.2 | 1.2 | 1.2 | 1.2 |
| Additives | Trishydroxymethylaminomethane | 0.053 | 0.053 | 0.053 | 0.053 |
| Dissolved oxygen concentration (mg/L) | | 2.85 | 3.45 | 4.95 | 9.3 |

(Evaluation)

Thermal Severe Test I was performed in the same manner as in Examples 1 to 14 and Comparative Example 2, and an evaluation of stability was performed.

The results are presented in Table 6. The amount of dimers after the Thermal Severe Test I can be adjusted to a preferable range by controlling the dissolved oxygen concentration to be 5 mg/L or less.

[Table 6]

| | Dimer amount (%) after Thermal Severe Test I |
|---|---|
| Example 15 | B(0.090) |
| Example 16 | B(0.095) |
| Example 17 | B(0.094) |
| Example 18 | B(0.096) |
| Example 19 | B(0.096) |
| Example 20 | B(0.100) |
| Example 21 | B(0.106) |
| Example 22 | B(0.130) |

(continued)

|  | Dimer amount (%) after Thermal Severe Test I |
|---|---|
| Comparative Example 3 | C(0.210) |

[Examples 23 to 27 and Comparative Example 4]

(Production of propofol-containing emulsion composition)

Propofol-containing oil-in-water emulsion compositions were produced by the following procedure, using the various components described in Table 7 at the contents described in the table (the numbers in the table represent w/v% with respect to the total capacity of the composition).

Various medium-chain fatty acid triglycerides were mixed with soybean oil, propofol was added thereto, and the mixture was stirred and dissolved. Thus, an oil phase was produced. Glycerin was dissolved in water, and an aqueous phase was produced. Purified egg yolk lecithin was added to the oil phase and mixed, and then the aqueous phase was added thereto. The resultant was subjected to rough emulsification using a HOMOMIXER (15,000 rpm (rotations/min), 5 minutes). This was passed 2 times through a high pressure emulsifying machine (STARBURST MINILABO, Sugino Corporation) under the conditions of 245 MPa. Subsequently, additives were added thereto, and the pH was adjusted to 8.0 using 1 mol/L hydrochloric acid or 1 mol/L sodium hydroxide. Thus, an emulsion composition was produced.

(Evaluation of filtering performance)

Samples of the emulsion compositions produced in Examples and Comparative Examples as described in Table 7, the samples being obtained before and after storage, were poured over a stainless steel pressure filter holder (47 mm, 340 mL, manufactured by Merck Millipore Corporation) with a predetermined filter for sterilization by filtration being mounted thereon, and the amount of liquid per unit time that had penetrated at a nitrogen pressure of 0.2 MPa was measured. The results thus obtained were used to calculate the filtration area needed to treat a liquid amount of 1 L according to a complete imperforation model of filtration, and a comparison was made. The evaluation criteria for the required filtration area are as follows.

A: Less than 200 cm$^2$/L
B: 200 cm$^2$/L or more and less than 1,000 cm$^2$/L
C: 1,000 cm$^2$/L or more

[Table 7]

|  |  | Example 23 | Example 24 | Example 25 |
|---|---|---|---|---|
| Oil phase | Propofol | 1 | 1 | 1 |
|  | MYGLYOL 812 | 5 | 5 | 5 |
|  | Japanese Pharmacopeia soybean oil | 1.5 | 2.5 | 0 |
| Aqueous phase | Japanese pharmacopeia glycerin | 2.25 | 2.25 | 2.25 |
|  | Water | Appropriate amount | Appropriate amount | Appropriate amount |
| Purified egg yolk lecithin | | 1.2 | 1.2 | 1.2 |
| Additives | Trishydroxymethylam inomethane | 0.053 | 0.053 | 0.053 |
| Material for filter for sterilization by filtration / trade name | | Cellulose acetate / Sartobran P (Sartorius Stedim Japan K.K.) | Cellulose acetate / Sartobran P (Sartorius Stedim Japan K.K.) | Cellulose acetate / Sartobran P (Sartorius Stedim Japan K.K.) |

|  |  | Example 26 | Example 27 | Comparative Example 4 |
|---|---|---|---|---|
| Oil phase | Propofol | 1 | 1 | 1 |
|  | MYGLYOL 812 | 5 | 5 | 5 |
|  | Japanese Pharmacopeia soybean oil | 1.5 | 1.5 | 5 |
| Aqueous phase | Japanese pharmacopeia glycerin | 2.25 | 2.25 | 2.25 |
|  | Water | Appropriate amount | Appropriate amount | Appropriate amount |
| Purified egg yolk lecithin | | 1.2 | 1.2 | 1.2 |
| Additives | Trishydroxymethylam inomethane | 0.053 | 0.053 | 0.053 |
| Material for filter for sterilization by filtration / trade name | | Polyvinylidene fluoride / Durapore (Merck Millipore AG) | Polyether sulfone / Sartopore 2 (Sartorius Stedim Japan K.K.) | Cellulose acetate / Sartobran P (Sartorius Stedim Japan K.K.) |

(Evaluation)

The results are presented in Table 8. When a filter for sterilization by filtration made of cellulose acetate is used, the required filtration area varies with the change in the particle size caused by composition change. When the particle size is 180 nm or less, preferable filtering performance is obtained. Filtering performance varies significantly depending on the type of filter, and filtering performance of a propofol-containing oil-in-water emulsion is obtained when a cellulose acetate filter is used.

[Table 8]

|  | Required filtration area (cm$^2$/L) | Average particle size (nm) |
|---|---|---|
| Example 23 | A(154.1) | 159.8 |
| Example 24 | B(411.1) | 175.5 |
| Example 25 | A(56.4) | 127.5 |
| Example 26 | B(579.8) | 134.6 |
| Example 27 | B(733.7) | 135.4 |
| Comparative Example 4 | C(1174.4) | 197 |

**Claims**

1. A propofol-containing oil-in-water emulsion composition comprising:

at least one selected from the group consisting of trishydroxymethylaminomethane, phosphoric acid, and triethanolamine;
propofol;
an oily component;
an emulsifier; and
water,
wherein a dissolved oxygen concentration is 5 mg/L or less and an average emulsion particle size is 180 nm or less.

2. The propofol-containing oil-in-water emulsion composition according to claim 1, which is charged into a plastic container.

3. The propofol-containing oil-in-water emulsion composition according to claim 1 or 2, wherein the at least one selected from the group consisting of trishydroxymethylaminomethane, phosphoric acid, and triethanolamine is trishydroxymethylaminomethane.

4. The propofol-containing oil-in-water emulsion composition according to any one of claims 1 to 3, further comprising an antioxidant,

wherein the antioxidant is at least one selected from the group consisting of citric acid or a salt thereof, ascorbic acid or a salt thereof, and a thiol-based antioxidant.

5. The propofol-containing oil-in-water emulsion composition according to claim 4, wherein the antioxidant is a thiol-based antioxidant.

6. The propofol-containing oil-in-water emulsion composition according to claim 4 or 5, wherein the trishydroxymethylaminomethane, and the thiol-based antioxidant and the citric acid or a salt thereof as antioxidants are included.

7. The propofol-containing oil-in-water emulsion composition according to claim 4 or 5, wherein the trishydroxymethylaminomethane, and the thiol-based antioxidant and the ascorbic acid or a salt thereof as antioxidants are included.

8. The propofol-containing oil-in-water emulsion composition according to any one of claims 4 to 7, wherein the thiol-based antioxidant is cysteine or a salt thereof, thioglycolic acid or a salt thereof, or thioglycerol.

9. The propofol-containing oil-in-water emulsion composition according to any one of claims 1 to 8, wherein the propofol at a proportion of 0.1 to 5 w/v%, the emulsifier at a proportion of 0.1 to 2 w/v%, and the oily component at a proportion of 0.1 to 9.9 w/v% are included.

10. A method for producing the propofol-containing oil-in-water emulsion composition according to any one of claims 1 to 9, the method comprising:

a step of treating a composition including propofol, an oily component, an emulsifier, and water using a high pressure homogenizer under the conditions of a pressure of 200 MPa or more; and

a step of sterilizing the composition thus obtained by passing the composition through a filter having a pore size of 0.22 $\mu$m or less.

11. The production method according to claim 10, wherein the material for the filter is cellulose acetate.

## INTERNATIONAL SEARCH REPORT

| International application No. |
| --- |
| PCT/JP2015/076870 |

### A. CLASSIFICATION OF SUBJECT MATTER
*A61K31/05*(2006.01)i, *A61K9/107*(2006.01)i, *A61K47/12*(2006.01)i, *A61K47/18*(2006.01)i, *A61K47/20*(2006.01)i, *A61K47/24*(2006.01)i, *A61P23/00*(2006.01)i

According to International Patent Classification (IPC) or to both national classification and IPC

### B. FIELDS SEARCHED

Minimum documentation searched (classification system followed by classification symbols)
A61K31/05, A61K9/107, A61K47/12, A61K47/18, A61K47/20, A61K47/24, A61P23/00

Documentation searched other than minimum documentation to the extent that such documents are included in the fields searched
Jitsuyo Shinan Koho        1922–1996    Jitsuyo Shinan Toroku Koho    1996–2015
Kokai Jitsuyo Shinan Koho   1971–2015    Toroku Jitsuyo Shinan Koho    1994–2015

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
| --- | --- | --- |
| Y/A | CN 102805728 A  (Nanjing Chia Tai Tianqing Pharmaceutical Co., Ltd.), 05 December 2012 (05.12.2012), entire text (Family: none) | 1,2,4,5,8–11 /6,7 |
| Y/A | JP 2004-526730 A  (Cydex, Inc.), 02 September 2004 (02.09.2004), claims; paragraphs [0003], [0005], [0091] to [0096], [0112] to [0113] & WO 2002/074200 A1 abstract; claims; page 1, lines 13 to 15; page 2, lines 8 to 10; page 25, line 24 to page 26, line 31; page 29, line 28 to page 30, line 9 & US 2003/0055023 A1    & EP 1383445 A1 & CA 2441744 A      & AU 2002254309 B & KR 10-2004-0097181 A | 1-5,8-11/6,7 |

[X] Further documents are listed in the continuation of Box C.          [ ] See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 26 November 2015 (26.11.15) | 08 December 2015 (08.12.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

**INTERNATIONAL SEARCH REPORT**

International application No.

PCT/JP2015/076870

C (Continuation).   DOCUMENTS CONSIDERED TO BE RELEVANT

| Category* | Citation of document, with indication, where appropriate, of the relevant passages | Relevant to claim No. |
|---|---|---|
| Y/A | JP 2012-210315 A  (Terumo Corp.),<br>01 November 2012 (01.11.2012),<br>abstract; claims; paragraph [0006]; examples<br>(Family: none) | 1-5,8-11/6,7 |
| Y/A | JP 2006-504771 A  (Transform Pharmaceuticals,<br>Inc.),<br>09 February 2006 (09.02.2006),<br>abstract; claims; paragraphs [0035], [0038];<br>examples<br>& WO 2004/039326 A2<br>abstract; claims; page 13, lines 8 to 18; page<br>14, lines 3 to 7; examples<br>& US 2004/0171691 A1    & EP 1555976 A2<br>& CA 2503956 A          & CN 1708270 A<br>& AU 2003286725 A | 1-5,8-11/6,7 |
| Y/A | US 6177477 B1  (George M.M.),<br>23 January 2001 (23.01.2001),<br>abstract; examples; claims<br>& WO 2000/056364 A1    & EP 1163007 A1<br>& DE 60001223 D        & AU 3909400 A<br>& BR 9286 A            & AT 231003 T<br>& CA 2367093 A        & DK 1163007 T<br>& ES 2189750 T | 1-5,8-11/6,7 |
| Y/A | WO 2006/112276 A1  (Otsuka Pharmaceutical<br>Factory, Inc.),<br>26 October 2006 (26.10.2006),<br>abstract; paragraphs [0029] to [0034], [0038]<br>to [0048]<br>& US 2009/0069445 A1<br>abstract; paragraphs [0037] to [0043], [0047]<br>to [0061]<br>& EP 1875901 A1        & KR 10-2008-0003860 A<br>& CN 101155580 A | 1-5,8-11/6,7 |
| Y/A | JP 2010-534555 A  (Shenyang Pharmaceutical<br>University),<br>11 November 2010 (11.11.2010),<br>abstract; claims; paragraphs [0002], [0030] to<br>[0034]; example 25<br>& US 2010/0189596 A1<br>abstract; claims; paragraphs [0002], [0029] to<br>[0038]; example 25<br>& WO 2009/012718 A1    & EP 2184100 A1<br>& CN 101091890 A | 1-5,8-11/6,7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005538191 A
- JP 2009504634 A
- JP 2010534555 A
- JP 2002179562 A